# EUROPÄISCHE PATENTANMELDUNG

(11) **EP 1 304 581 A2**
(43) Veröffentlichungstag der Anmeldung: **23.04.2003**
(21) Anmeldenummer: 02102455.9
(22) Anmeldetag: 17.10.2002
(51) Int. Cl.: G01R 33/28, A61B 5/06

(54) **Verfahren zum Lokalisieren eines Gegenstandes in einer MR-Apparatur sowie Katheter und MR-Apparatur zur Durchführung des Verfahrens**

(30) Priorität: 19.10.2001 DE 10151779
(71) Anmelder: Philips Corporate Intellectual Property GmbH, 52064 Aachen (DE); Koninklijke Philips Electronics N.V., 5621 BA Eindhoven (NL)
(72) Erfinder: Gleich, Bernhard, c/o Philips Corp.Int.Prop.GmbH, 52088, Aachen (DE)
(74) Vertreter: Volmer, Georg, Dipl.-Ing.

(57) **Zusammenfassung**

Bei einem Verfahren zum Lokalisieren eines Gegenstandes, vorzugsweise eines medizinischen Instruments, insbesondere eines Katheters (60), welcher bzw. welches sich in einem Körper im Untersuchungsraum einer nach der Methode der Kernspinresonanz bzw. Magnetresonanz (MR) arbeitenden Apparatur befindet, wird die Wechselwirkung eines an dem Gegenstand (60) angebrachten elektromagnetischen Resonanzkreises (6) mit einem in der MR-Apparatur zur Kernmagnetisierung im Körper angelegten HF-Feld ausgewertet

Bei einem solchen Verfahren wird eine einfache, schnelle und genaue Lokalisierung eines Gegenstandes, insbesondere eines Katheters, in einer bildgebenden MR-Einrichtung dadurch ermöglicht, daß ein auf die Frequenz des HF-Feldes abgestimmter Resonanzkreis (6) verwendet wird, welcher zwei Zustände unterschiedlicher Resonanzkreisgüte einnehmen kann, daß in einem ersten Schritt mittels eines ersten HF-Pulses eine Kernmagnetisierung mit einem Flipwinkel hervorgerufen wird, wobei sich der Resonanzkreis (6) im einen der beiden Zustände befindet, und daß in einem zweiten Schritt mittels eines zweiten HF-Pulses die Kernmagnetisierung zurückgeklappt wird, wobei sich der Resonanzkreis (6) im anderen der beiden Zustände befindet.

## Beschreibung

Die vorliegende Erfindung bezieht sich auf das Gebiet der Kernspinresonanz-Tomographie. Sie betrifft ein Verfahren zum Lokalisieren eines Gegenstandes, insbesondere eines Katheters, welcher sich in einem Körper im Untersuchungsraum einer nach der Methode der Kernspinresonanz bzw. Magnetresonanz (MR) arbeitenden Apparatur befindet, gemäß dem Oberbegriff des Anspruchs 1. Sie betrifft weiterhin ein Katheter und eine MR-Apparatur zur Durchführung des Verfahrens gemäß dem Oberbegriff des Anspruchs 8 bzw. des Anspruchs 10.

Ein solches Verfahren und solche Vorrichtungen sind z.B. aus der EP-A2-0 928 972 bekannt.

Bildgebende Magnetresonanz(MR)-Verfahren (Magnetic Resonance Imaging MRI), welche die Wechselwirkung von Magnetfeldern mit Kernspins zur Erstellung von zweidimensionalen oder dreidimensionalen Bildern ausnutzen, haben insbesondere in der medizinischen Diagnostik eine verbreitete Anwendung gefunden, weil sie bei der Darstellung von Weichteilstrukturen anderen bildgebenden Verfahren in vielen Bereichen überlegen sind, ohne ionisierende Strahlung auskommen und in der Regel nicht invasiv sind.

Bei den MR-Verfahren wird der zu untersuchende Körper in ein starkes homogenes Magnetfeld gebracht, dessen Richtung zugleich eine Achse (üblicherweise die z-Achse) des der Messung zugrundeliegenden Koordinatensystems definiert. Durch das Magnetfeld werden für die einzelnen Kernspins in Abhängigkeit von der Magnetfeldstärke unterschiedliche energetische Niveaus erzeugt, die durch Einstrahlung eines elektromagnetischen Wechselfeldes definierter Frequenz (Larmorfrequenz) angeregt werden können (Kernspinresonanz).

Makroskopisch gesehen ergibt die Verteilung der einzelnen Kernspins eine Gesamtmagnetisierung, die durch einen eingestrahlten elektromagnetischen Puls entsprechender Frequenz (HF-Puls), bei dem das Magnetfeld senkrecht zur z-Achse steht, auf einer spiralförmigen Bahn aus der Gleichgewichtslage ausgelenkt werden kann und dann eine Präzessionsbewegung um die z-Achse ausführt. Die Präzessionsbewegung beschreibt einen Kegelmantel, dessen Öffnungswinkel als Flipwinkel bezeichnet wird. Die Größe des Flipwinkels hängt von der Stärke und Dauer des eingestrahlten elektromagnetischen Pulses ab. Bei einem sogenannten 90°-Puls werden die Spins aus der z-Achse in die Transversalebene geklappt (Flipwinkel 90°).

Nach dem Ende des HF-Pulses relaxiert die Magnetisierung wieder in die ursprüngliche Gleichgewichtslage zurück, wobei sich mit einer ersten Zeitkonstanten T₁ (Spin-Gitter-Relaxationszeit) die Magnetisierung in z-Richtung wieder aufbaut, und mit einer zweiten Zeitkonstanten T₂ (Spin-Spin-Relaxationszeit) die Magnetisierung senkrecht zur z-Richtung abbaut. Die Änderung in der Magnetisierung kann mit einer Spule detektiert werden, die üblicherweise so orientiert ist, daß die Änderung der Magnetisierung senkrecht zur z-Achse (Transversalmagnetisierung, Zeitkonstante T₂) gemessen wird. Mit dem Abbau der Transversalmagnetisierung verbunden ist nach Anlegung eines 90°-Pulses ein durch lokale Magnetfeldinhomogenitäten bewirkter Übergang der Kernspins von einem geordneten Zustand gleicher Phase in einen Gleichgewichtszustand, bei dem alle Phasenwinkel gleich verteilt sind (Dephasierung). Die Dephasierung kann durch einen Refokussierungspuls (180°-Puls) kompensiert werden. Hierdurch ergibt sich in der Detektionsspule ein Echosignal (Spinecho).

Um im Körper eine Ortsauflösung zu erhalten, werden dem homogenen Magnetfeld lineare Gradientenfelder in den drei Hauptachsen überlagert, die zu einer linearen Ortsabhängigkeit der Kernspinresonanzfrequenz führen. Das in der Detektionsspule aufgenommene Signal enthält dann Anteile verschiedener Frequenzen, die nach einer Fouriertransformation von der Zeit- auf die Frequenzachse verschiedenen Orten im Körper zugeordnet werden können.

Ein derartiges bildgebendes MR-Verfahren kann gleichzeitig dazu eingesetzt werden, ein in den Körper eingeführtes medizinisches Instrument, insbesondere einen Katheter, zu lokalisieren bzw. seine Bewegung im Körper zu verfolgen.

Es ist deshalb vorgeschlagen worden (siehe die EP-A2-0 928 972), an der Instrumentenspitze zur Lokalisierung des Instruments (Katheters) einen geschlossenen, aus Mikrospule und Kondensator gebildeten Resonanzkreis anzuordnen. Der auf die beim MR-Verfahren auftretenden Frequenzen abgestimmte Resonanzkreis erhöht durch seine Resonanzeigenschaften lokal das HF-Signal und damit den Flipwinkel im Körper. Diese lokale Erhöhung kann bei der MR-Bilderzeugung detektiert und zur Lokalisierung der Instrumentenspitze verwendet werden. Problematisch ist bei diesem Verfahren jedoch, daß die Lokalisierung der Instrumentenspitze Teil der Bilderzeugung ist und damit vergleichsweise langsam vonstatten geht. Darüber hinaus erschwert die Überlagerung des durch den Resonanzkreis verstärkten HF-Signals und der im übrigen Teil des Körpers auftretenden HF-Signale die Detektion und Lokalisierung.

Eine andere Lösung zur Lokalisierung (DE-A1-199 56 595) setzt einen nichtlinearen Resonanzkreis mit einer Mikrospule ein. Die Anregung der Kernmagnetisierung wird mit einem HF-Puls vorgenommen, dessen Frequenzspektrum nicht mit dem Bereich des Kernresonanzspektrums überlappt, so daß außerhalb des Nahbereichs der Mikrospule eine Kernmagnetisierung nicht angeregt wird. Der nichtlineare Resonanzkreis erzeugt jedoch aufgrund seiner Nichtlinearität aus dem HF-Puls lokal ein mit der Kernresonanzfrequenz überlappendes HF-Signal, das lokal eine Anregung der Kernmagnetisierung bewirkt. Diese lokale Kernmagnetisierung kann vermessen und die ermittelte Position in das MR-Bild des untersuchten Körpers eingeblendet werden.

Nachteilig ist bei diesem Verfahren, daß mit einer anderen als der Resonanzfrequenz (Larmorfrequenz) gearbeitet werden muß, und daß vor allem die Erzeugung der Larmorfrequenz in Form von höheren Harmonischen durch den nichtlinearen Resonanzkreis nur wenig effektiv ist und geringe Feldstärken produziert.

Es ist daher Aufgabe der Erfindung, ein Verfahren, ein Katheter und eine MR-Apparatur anzugeben, mit welchen eine einfache, schnelle und genaue Lokalisierung in einem zu untersuchenden Körper ermöglicht wird.

Die Aufgabe wird durch die der Merkmale der Ansprüche 1, 8 bzw. 10 gelöst.

Der Kern der Erfindung besteht darin, an dem zu lokalisierenden Katheter einen zuleitungsfreien Resonanzkreis anzubringen, der unterschiedliche Resonanzgüten aufweisen kann. Wird die Kernmagnetisierung durch einen ersten HF-Puls angeregt und durch einen zweiten nachfolgenden HF-Puls wieder zurückgeklappt, wobei der Resonanzkreis beim ersten und zweiten HF-Puls eine unterschiedliche Resonanzgüte aufweist, bleibt außerhalb der zum Resonanzkreis gehörenden Spule kein Nettoeffekt, während im engen Umfeld der Spule durch die unterschiedliche Resonanzüberhöhung des Feldes eine Nettomagnetisierung verbleibt, die zur Ortsbestimmung in der MR-Apparatur ausgewertet werden kann. Durch diese Art der Differenzmessung kann eine schnelle und präzise Echtzeitlokalisierung erreicht werden, die ohne Aufbau eines vollständigen Bildes auskommt, für den Patienten sicher ist, leicht zu verwirklichen ist und keinen zusätzlichen Apparateaufwand benötigt. Darüber hinaus ist die Lokalisierung wegen der Schnelligkeit weitgehend frei von Bewegungseinflüssen.

Gemäß einer bevorzugten Ausgestaltung des erfindungsgemässen Verfahrens ist der Resonanzkreis zwischen den beiden Zuständen umschaltbar, und wird der Resonanzkreis im Verlauf der Lokalisierung zwischen den beiden Zuständen umgeschaltet. Hierdurch wird eine eindeutige und gut auswertbare Differenzbildung ermöglicht.

Bevorzugt wird der Resonanzkreis durch einen HF-Puls umgeschaltet, wobei zum Umschalten insbesondere einer der zwei für die Kernmagnetisierung bzw. deren Zurückklappen benötigten HF-Pulse verwendet wird. Dies hat den Vorteil, daß keine zusätzlichen Einrichtungen zur Beeinflussung des Resonanzkreises benötigt werden.

Eine bevorzugte Weiterbildung dieser Ausgestaltung ist dadurch gekennzeichnet, daß zur Kernmagnetisierung und deren Zurückklappen zwei HF-Pulse verwendet werden, von denen der eine eine niedrige Leistung und eine lange Pulsdauer und der andere eine hohe Leistung und eine kurze Pulsdauer aufweisen, und daß der HF-Puls mit der hohen Leistung und der kurzen Pulsdauer zum Umschalten des Resonanzkreises verwendet wird.

Eine sehr einfache Realisierung der Erfindung ergibt sich, wenn zum Umschalten ein im Resonanzkreis angeordnetes nichtlineares Element verwendet wird.

Es ist aber auch denkbar, daß der Resonanzkreis ein optisch steuerbares Element enthält und durch ein optisches Signal umschaltet wird.

Gemäß einer bevorzugten Ausgestaltung des Katheters nach der Erfindung ist das Schaltelement eine Diode, und zwar entweder eine einfache Diode, welche der Kapazität parallelgeschaltet ist, oder eine Kapazitätsdiode, welche mit der Kapazität in Serie geschaltet ist, oder eine Photodiode, wobei eine Serienschaltung der Photodiode und einer weiteren Kapazität der Kapazität parallelgeschaltet ist.

Weitere Ausgestaltungen ergeben sich aus den abhängigen Ansprüchen.

Die Erfindung soll nachfolgend anhand von Ausführungsbeispielen im Zusammenhang mit der Zeichnung näher erläutert werden. Es zeigen
- Fig. 1: eine stark vereinfachte Darstellung einer MR-Apparatur, mit welcher das erfindungsgemässe Verfahren durchführbar ist;
- Fig. 2: das Prinzipschaltbild einer solchen Apparatur;
- Fig. 3: das Schaltbild und die Anordnung in einem Katheter eines Resonanzkreises gemäß einem ersten Ausrührungsbeispiel der Erfindung;
- Fig. 4: eine beispielhafte Abfolge von HF-Pulsen zur Lokalisierung gemäß dem Verfahren nach der Erfindung; und
- Fig. 5: das Schaltbild eines Resonanzkreises gemäß einem zweiten Ausführungsbeispiel der Erfindung.

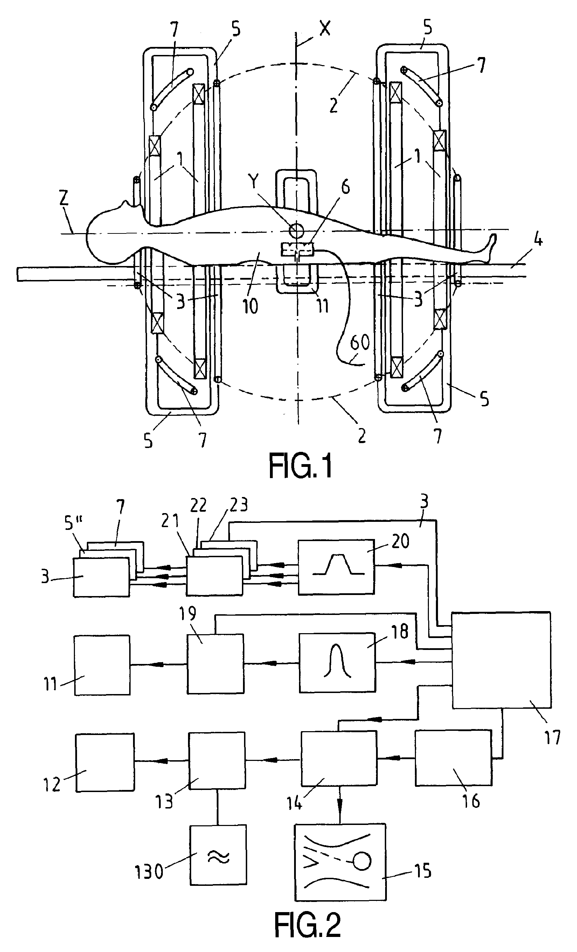

In den Fig. 1 und 2 ist eine MR-Apparatur dargestellt, mit welcher das erfindungsgemässe Verfahren durchgeführt werden kann. Die in Fig. 1 dargestellte Vorrichtung zur Erzeugung von MR-Bildern, die auch als Kernspinuntersuchungsgerät bezeichnet wird, weist eine aus vier Hauptspulen 1 bestehende Anordnung zur Erzeugung eines homogenen stationären Magnetfeldes in z-Richtung (Hauptfeld) auf, dessen magnetische Flussdichte (magnetische Induktion) in der Größenordnung von einigen Zehntel Tesla bis einigen Tesla liegen kann. Die zu der z-Achse konzentrisch angeordneten Hauptspulen 1 können auf einer Kugeloberfläche 2 liegen. Im Innern dieser Spulen befindet sich ein Untersuchungsobjekt, zum Beispiel ein Patient 10 auf einer Tischplatte 4.

Zur Erzeugung eines in Richtung der z-Achse verlaufenden und sich in dieser Richtung linear ändernden ersten Gradienten-Magnetfeldes sind vier erste Spulen 3 auf der Kugeloberfläche 2 oder einer Zylinderoberfläche angeordnet. Weiterhin sind vier zweite Spulen 7 vorgesehen, die ein ebenfalls in Richtung der z-Achse verlaufendes zweites Gradienten-Magnetfeld erzeugen, das sich jedoch in vertikaler Richtung (x-Richtung) linear ändert. Schließlich wird mit vier dritten Spulen 5 (von denen nur zwei dargestellt sind) ein in Richtung der z-Achse verlaufendes, drittes Gradient-Magnetfeld erzeugt, das sich senkrecht zur Zeichenebene der Fig. 1 (y-Richtung) linear ändert.

In den zu untersuchenden Bereich des Patienten ist ein medizinisches Instrument (zum Beispiel ein Katheter) 60 eingeführt, an dessen Spitze sich ein Resonanzkreis 6 befindet. Dieser Bereich ist ferner von einer durch einen HF-Impuls beaufschlagbaren Hochfrequenz-Sendespule 11 umgeben, durch die dieser Bereich mit einem eine Spinresonanz anregenden HF-Magnetfeld durchsetzt wird. Die sich an diese Anregung anschließende Relaxation bewirkt eine Änderung der Magnetisierungszustände, die in einer Hochfrequenz-Empfangsspule 12 (siehe Fig. 2) eine entsprechende Spannung induziert, die zur MR-Bilderzeugung ausgewertet wird, wobei die Gradienten-Magnetfelder eine Lokalisierung der angeregten Zustände ermöglichen.

Die zum Betrieb dieser Vorrichtung wesentlichen Komponenten sind schematisch in Fig. 2 dargestellt und umfassen eine Steuereinheit 17, die einen Gradienten-Wellenform-Generator 20 ansteuert, an dessen Ausgängen jeweils ein erster, ein zweiter und ein dritter Gradientenverstärker 21, 22, 23 angeschlossen ist. Diese Verstärker erzeugen jeweils den Strom für die erste, zweite bzw. dritte Spule 3, 5, 7. Die Verstärkungsfaktoren dieser Verstärker sind durch die Steuereinheit 17 über Leitungen 39 unabhängig voneinander einstellbar, so daß die Spulen 3, 5, 7 die Gradientfelder in den x-, y-und z-Richtungen erzeugen und eine Schichtselektion in den entsprechenden drei Raumrichtungen in dem untersuchten Bereich vorgenommen werden kann.

Weiterhin wird durch die Steuereinheit 17 ein HF-Generator 18 angesteuert, um zur MR-Bilderzeugung die Frequenz der HF-Pulse auf die von den Gradientenfeldern abhängigen Larmor-Frequenzen abzustimmen und HF-Pulse unterschiedlicher Länge zu erzeugen. Die HF-Impulse werden einem Verstärker 19 zugeführt, dessen Verstärkung durch die Steuereinheit 17 gesteuert wird, und gelangen anschließend zu der Hochfrequenz-Sendespule 11.

Die in der Hochfrequenz-Empfangsspule 12 durch die Relaxation der angeregten Magnetisierungszustände induzierten MR-Signale werden in einem Quadratur-Demodulator 13 durch Mischung mit zwei um 90° gegeneinander versetzten Trägerschwingungen (mit einer durch die lokale Stärke der stationären Magnetfelder bestimmten Larmor- bzw. MR-Frequenz) eines Oszillators 130 demoduliert, so daß zwei Signale entstehen, die als Realteil und als Imaginärteil eines komplexen Signals aufgefaßt werden können. Diese Signale werden einem Analog-Digitalwandler 14 zugeführt. Mit einer Bildverarbeitungseinheit 16 werden schließlich die MR-Bilder in bekannter Weise rekonstruiert und auf einem Monitor 15 wiedergegeben.

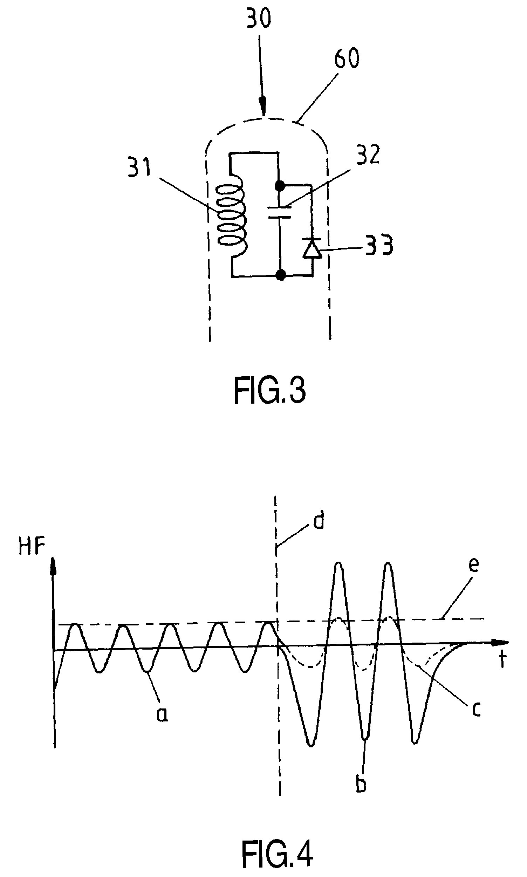

Der Resonanzkreis 6 aus Fig. 1, der ohne elektrische Zuleitungen nach außen auskommt, kann unterschiedliche Formen annehmen: In Fig. 3 ist das Schaltbild eines Resonanzkreises 30 wiedergegeben, der in der Spitze eines (gestrichelt eingezeichneten) Katheters 60 angeordnet ist. Der Katheter 60 wird in den Körper einer zu untersuchenden Person (Patient 10) eingeführt, die sich gemäß Fig. 1 im Untersuchungsraum der dort gezeigten MR-Apparatur befindet. Der Resonanzkreis 30 umfaßt eine Induktivität 31, die vorzugsweise in Form einer Mikrospule vorliegt, sowie eine dazu parallelgeschaltete Kapazität 32. Induktivität 31 und Kapazität 32 bilden einen Parallelschwingkreis, der im wesentlichen auf die Larmorfrequenz des durch die MR-Apparatur angeregten Körpermaterials abgestimmt ist. Parallel zur Kapazität 32 ist als nichtlineares Element eine Diode 33 angeordnet.

Der Resonanzkreis 30 koppelt an einen von der MR-Apparatur zur Anregung der Kernmagnetisierung ausgesandten HF-Puls an. Wenn die HF-Leistung des Pulses klein ist, ist auch die Spannung an der Diode 33 klein. Die Diode 33 leitet dann nicht, weil ihre Schwellenspannung nicht überschritten wird. Die Resonanzkreisgüte (Schwingkreisgüte) des Resonanzkreises (Schwingkreises) 30 ist dann vergleichsweise hoch und das lokale HF-Feld im Bereich der Mikrospule 31 wird dann mit einem Faktor G1>> 1 multipliziert. Wenn die HF-Leistung deutlich erhöht wird, wird die Diode 33 leitend und verringert durch die damit verbundene Bypassfunktion die Resonanzkreisgüte: Das lokale HF-Feld wird nicht so stark erhöht; der Multiplikationsfaktor nimmt dann einen Wert G2 < G1 ein.

Dieses Verhalten des Resonanzkreises 30 kann zu Realisierung einer Differenzmethode herangezogen werden, wobei das Verhalten des Resonanzkreises 30 anhand des Verlaufes des lokalen HF-Feldes (HF) über der Zeit (t) in Fig. 4 schematisch dargestellt ist. Wenn in einem ersten Schritt ein längerer HF-Puls relativ niedriger HF-Leistung benutzt wird, um die Magnetisierung im Bereich des Katheters 60 umzukippen, wird der Flipwinkel an der Spitze des Katheters 60, d.h., im Bereich der Mikrospule 31, durch die Resonanzüberhöhung deutlich vergrößert (Faktor G1; Kurve a in Fig. 4; ein bei Kurve e einsetzendes Clipping durch Diode 33 findet nicht statt). Wenn dann (ab Grenzlinie d in Fig. 4) ein zweiter kurzer HF-Puls (Kurve b in Fig. 4) mit um 180° gedrehter Phase und relativ hoher HF-Leistung angelegt wird, um die Magnetisierung zurückzuklappen, wird die angeregte Magnetisierung im Bereich außerhalb der Mikrospule 31 Null, wenn die Zeitintegrale der HF-Pulse gleich sind. In unmittelbarer Nähe der Mikrospule 31 ist die Magnetisierung dagegen ungleich Null, weil der Effekt der Resonanzüberhöhung durch das Clipping-Verhalten der Diode 33 geringer ist (Faktor G2; das durch die Diode begrenzte HF-Feld verläuft entlang der Kurve c in Fig. 4).

In einer Projektion zeigt sich dann die Spitze des Katheters 60 als ein einzelner Peak. Unter einer Projektion wird dabei Folgendes verstanden: Durch die HF-Pulse wird ein Volumen (im Untersuchungsraum) angeregt. Zu dem Zeitpunkt, an dem das Echo (Spinecho) auftritt, wird ein Magnetfeldgradient in einer Projektionsrichtung angelegt. Durch Fouriertransformation des erhaltenen Signals bekommt man eine Projektion, d.h., entlang der Ortskoordinate ist die Signalintensitätsverteilung aufgetragen, die sich aus der Integration der Schichten senkrecht zur Projektionsrichtung ergibt. Wenn in solch einer Projektion ein klarer, durch den Katheter 60 verursachter Peak zu entdecken ist, hat man durch diese Messung seine Position in einer Raumrichtung. Durch insgesamt drei Messungen in drei orthogonalen Raumrichtungen bekommt man die Position des Katheters 60 im Raum. Diese Lokalisierung durch Projektion ist sehr schnell. Würde dagegen die Lokalisierung des Katheters 60 durch Aufnahme eines vollständigen Bildes erfolgen, benötigte man 256 oder mehr dieser Schritte (für den Aufbau eines kompletten Bildes) und könnte dann noch nicht einmal sicher sein, den Katheter überhaupt zu erfassen.

Es ist auch möglich, bei der Lokalisierung durch Anlegen eines Magnetfeldgradienten eine bestimmte Schicht des Untersuchungsraumes auszuwählen, um bei Anwesenheit mehrerer markierter Instrumente die einzelnen Instrumente mit ihren jeweiligen Resonanzkreisen (Markern) unterscheiden zu können. Bevor der zweite HF-Puls zum Zurückklappen der Magnetisierung angelegt wird, muß dann allerdings in an sich bekannter Weise eine Refokussierung der Spins durch einen Refokussierungspuls erfolgen.

Wird kein bestimmtes Volumen des Untersuchungsraumes ausgewählt, umfaßt die Messsequenz folgende Schritte:
- HF-Puls mit Leistung 1;
- HF-Puls mit Leistung 2, der die Magnetisierung entfernt vom Katheter zurückklappt;
- ggf. Refokussierungspulse bei Spinecho;
- Gradient in Projektionsrichtung;
- Datenaufnahme.

Wird dagegen ein bestimmtes Volumen des Untersuchungsraumes ausgewählt, ergibt sich die folgende Messsequenz:
- Gradientenlokalisierungssequenz mit HF-Puls mit Leistung 1 (mit oder ohne Refokussierung, je nach Zeitverlauf);
- HF-Puls mit Leistung 2, der die Magnetisierung entfernt vom Katheter zurückklappt; dabei wird eine Gradientensequenz gefahren, die das gleiche Volumen selektiert;
- ggf. Refokussierungspuls bei Spinecho;
- Gradient in Projektionsrichtung;
- Datenaufnahme.

Weiterhin ist es möglich, anstelle der Diode 33 andere nichtlineare Bauelemente im Resonanzkreis 30 einzusetzen: Vorstellbar sind hier Halbleiterbauelemente mit mehreren PN-Übergängen wie z.B. Transistoren, Feldeffektbauelemente (FETs, bei denen Source und Gate verbunden sind), oder sogenannte Wollastondrähte, d.h., extrem dünne Drähte aus Platin oder dgl., die sich bei Strombelastung extrem schnell aufheizen und damit ihren Widerstand vergrößern. Denkbar sind auch verschiedene Thermowiderstände (PTC, NTC), wenn sie kleine genug gebaut sind, sowie Kondensatoren mit sättigbaren Dielektrika (Ferroelektrika). Dioden sind jedoch bei weitem am besten geeignet.

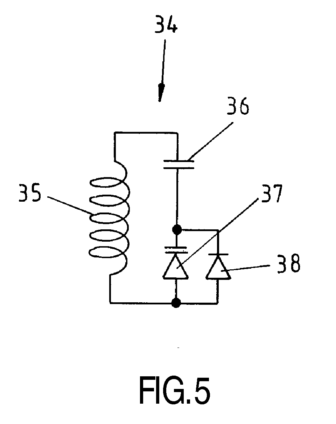

Wenn eine Störung des in der MR-Apparatur erzeugten Bildes durch die Marker nicht gewünscht ist, kann ein alternativer Resonanzkreis verwendet werden, der durch einen kurzen HF-Puls hoher Leistung in den Zustand mit großer Resonanzüberhöhung umgeschaltet wird. Ein Ausrührungsbeispiel für einen solchen Resonanzkreis ist in Fig. 5 wiedergegeben. Der Resonanzkreis 34 der Fig. 5 umfaßt wiederum eine Mikrospule 35 und eine Kapazität 36 in Parallelschaltung. In Serie mit der Kapazität liegt innerhalb der Parallelschaltung eine Kapazitätsdiode (Varicapdiode) 37, der eine weitere Diode 38 parallelgeschaltet ist. Die Diode 38 sollte dabei eine möglichst geringe Durchlassspannung haben. Im Ruhezustand hat die Kapazitätsdiode 37 eine hohe Kapazität und die Resonanzfrequenz des Resonanzkreises 34 liegt tief. Durch einen starken HF-Puls (in diesem Fall auch ein Puls, der eine niedrige Frequenz hat) wird die Kapazitätsdiode 37 geladen und verkleinert ihre Kapazität. Die Resonanzfrequenz des Resonanzkreises 34 steigt. Dieser Zustand bleibt eine kurze Zeit erhalten.

Die beschriebene Differenzmethode, bei der zunächst einen ersten HF-Puls die Magnetisierung umgeklappt und dann durch einen zweiten HF-Puls zurückgeklappt wird, ist für alle Marker geeignet, die zwischen den Pulsen hinsichtlich der Beeinflussung der Magnetisierung umgeschaltet werden können. Ein Beispiel für einen weiteren umschaltbaren Marker ist ein optisch umschaltbarer Resonanzkreis. Ein solcher Resonanzkreis hat einen zu Fig. 3 vergleichbaren Aufbau, mit dem Unterschied, daß die Diode 33 durch eine Photodiode ersetzt ist. Über einen Lichtleiter wird dann zwischen den HF-Pulsen ein optischer Impuls in den Katheter 60 eingeleitet, der die Photodiode leitend macht und damit die Resonanzkreisgüte herabsetzt.

Der Vorteil der Differenzmethode ist allgemein, daß bewegungsbedingte Einflüsse auf das MR-Bild weitgehend wegfallen, weil die Differenzbildung innerhalb einer Millisekunde abgeschlossen ist.

Insgesamt ergeben sich mit der Erfindung die folgenden Vorteile:
- die Lokalisierung ist präzise;
- die Lokalisierung ist in Echtzeit möglich;
- die Methode ist HF-sicher für einen Patienten;
- die Marker sind leicht aufzubauen und es werden in der MR-Apparatur keine zusätzlichen Einrichtungen benötigt;
- in Differenzbild gibt es keine bewegungsbedingten Störungen.

### BEZUGSZEICHENLISTE

- 1: Hauptspule
- 2: Kugeloberfläche
- 3,5,7: Spule
- 4: Tischplatte
- 6,30,34: Resonanzkreis
- 10: Patient
- 11: Hochfrequenz-Sendespule
- 12: Hochfrequenz-Empfangsspule
- 13: Quadratur-Demodulator
- 14: Analog-Digitalwandler
- 15: Monitor
- 16: Bildverarbeitungseinheit
- 17: Steuereinheit
- 18: HF-Generator
- 19: Verstärker
- 20: Gradienten-Wellenform-Generator
- 21,22,23: Gradientenverstärker
- 31,35: Induktivität
- 32,36: Kapazität
- 33,38: Diode
- 37: Kapazitätsdiode
- 39: Leitung
- 60: Katheter (medizinisches Instrument)
- 130: Oszillator
- a,..,e: Kurve

## Patentansprüche

1. Verfahren zum Lokalisieren eines Gegenstandes, vorzugsweise eines medizinischen Instruments, insbesondere eines Katheters (60), welcher bzw. welches sich in einem Körper im Untersuchungsraum einer nach der Methode der Kernspinresonanz bzw. Magnetresonanz (MR) arbeitenden Apparatur befindet, bei welchem Verfahren die Wechselwirkung eines an dem Gegenstand (60) angebrachten, zuleitungsfreien elektromagnetischen Resonanzkreises (30, 34) mit einem in der MR-Apparatur zur Kernmagnetisierung im Körper angelegten HF-Feld ausgewertet wird,
**dadurch gekennzeichnet,**
**dass** ein auf die Frequenz des HF-Feldes abgestimmter Resonanzkreis (30, 34) verwendet wird, welcher zwei Zustände unterschiedlicher Resonanzkreisgüte einnehmen kann, daß in einem ersten Schritt mittels eines ersten HF-Pulses eine Kernmagnetisierung mit einem Flipwinkel hervorgerufen wird, wobei sich der Resonanzkreis (30, 34) im einen der beiden Zustände befindet, und daß in einem zweiten Schritt mittels eines zweiten HF-Pulses die Kernmagnetisierung zurückgeklappt wird, wobei sich der Resonanzkreis (30, 34) im anderen der beiden Zustände befindet.

2. Verfahren nach Anspruch 1,
**dadurch gekennzeichnet,**
**dass** der Resonanzkreis (30, 34) zwischen den beiden Zuständen umschaltbar ist, und daß der Resonanzkreis (30, 34) im Verlauf der Lokalisierung zwischen den beiden Zuständen umgeschaltet wird.

3. Verfahren nach Anspruch 2,
**dadurch gekennzeichnet,**
**dass** der Resonanzkreis (30, 34) durch einen HF-Puls umgeschaltet wird, und daß zum Umschalten einer der zwei für die Kernmagnetisierung bzw. deren Zurückklappen benötigten HF-Pulse verwendet wird.

4. Verfahren nach Anspruch 3,
**dadurch gekennzeichnet,**
**dass** zur Kernmagnetisierung und deren Zurückklappen zwei HF-Pulse verwendet werden, von denen der eine eine niedrige Leistung und eine lange Pulsdauer und der andere eine hohe Leistung und eine kurze Pulsdauer aufweisen, und daß der HF-Puls mit der hohen Leistung und der kurzen Pulsdauer zum Umschalten des Resonanzkreises (30, 34) verwendet wird.

5. Verfahren nach Anspruch 4,
**dadurch gekennzeichnet,**
**dass** zum Umschalten ein im Resonanzkreis (30, 34) angeordnetes nichtlineares Element (33, 37) verwendet wird.

6. Verfahren nach Anspruch 4,
**dadurch gekennzeichnet,**
**dass** als erster HF-Puls der HF-Puls mit der niedrigen Leistung und der langen Pulsdauer angelegt wird, daß als zweiter HF-Puls der HF-Puls mit der hohen Leistung und der kurzen Pulsdauer angelegt wird, und daß der zweite HF-Puls den Resonanzkreis (30) vom Zustand hoher Resonanzkreisgüte in den Zustand niedriger Resonanzkreisgüte umschaltet.

7. Verfahren nach Anspruch 2,
**dadurch gekennzeichnet,**
**dass** der Resonanzkreis ein optisch steuerbares Element enthält und durch ein optisches Signal umschaltet wird.

8. Katheter (60) zur Durchführung des Verfahrens nach Anspruch 1, welcher Katheter (60) einen zuleitungsfreien Resonanzkreis (30, 34) umfaßt, der eine Induktivität (31, 35), insbesondere in Form einer Mikrospule, und eine der Induktivität (31, 35) parallel geschaltete Kapazität (32, 36) enthält,
**dadurch gekennzeichnet,**
**dass** in dem Resonanzkreis (30, 34) ein zusätzliches Schaltelement (33, 37) angeordnet ist, welches die Resonanzkreisgüte des Resonanzkreises (30, 34) beeinflußt.

9. Katheter nach Anspruch 8,
**dadurch gekennzeichnet,**
**dass** das zusätzliche Schaltelement eine Diode (33, 37) ist.

10. MR-Apparatur zur Durchführung des Verfahrens nach Anspruch 1, mit ersten Mitteln (1) zur Erzeugung eines homogenen stationären Magnetfeldes, dessen Stärke die Lamorfrequenz definiert, mit zweiten Mitteln (11, 18, 19) zur Erzeugung von HF-Pulsen und dritten Mitteln (12, 13, 14, 16) zum Empfangen von im Untersuchungsobjekt (10) erzeugten MR-Signalen, **gekennzeichnet durch** eine Steuereinheit (17) zum Steuern der HF-Pulse in der Weise, dass in einer zeitlichen Abfolge ein erster HF-Puls mit einer ersten Amplitude und einer ersten Pulsdauer zur Kernmagnetisierung mit einem Flipwinkel und ein zweiter HF-Puls mit einer zweiten Amplitude und zweiten Pulsdauer zum Zurückklappen der Kemmagnetisierung erzeugt wird.
